# EUROPEAN PATENT SPECIFICATION

(11) **EP 1 885 336 B1**
(45) Date of publication and mention of the grant of the patent: **15.04.2009**
(21) Application number: 06759258.4
(22) Date of filing: 08.05.2006
(51) Int. Cl.: A61K 9/00, A61K 31/165, A61K 31/542, A61K 31/57

(54) **SUSPENSION FORMULATIONS COMPRISING AN ACTIVE PRINCIPLE, A POLOXAMER OR MEROXAPOL SURFACTANT AND A GLYCOL, ITS USE FOR THE MANUFACTURE OF A MEDICAMENT FOR TREATING OPHTHALMIC DISORDERS**
SUSPENSIONSFORMULIERUNGEN MIT EINEM AKTIVEN WIRKSTOFF, EINEM POLOXAMER- ODER MEROXAPOL-TENSID UND EINEM GLYCOL UND IHRE VERWENDUNG ZUR HERSTELLUNG EINES MEDIKAMENTS ZUR BEHANDLUNG VON AUGENKRANKHEITEN
FORMULATION DE SUSPENSIONS CONTENANT UN PRINCIPE ACTIF, UN AGENT TENSIOACTIF DE TYPE POLOXAMERE OU MEROXAPOL ET UN GLYCOL, LEUR EMPLOI DANS LA PRODUCTION D'UN MEDICAMENT POUR TRAITER LES MALADIES OPHTALMIQUES

(30) Priority: 10.05.2005 US 679332 P
(43) Date of publication of application: 13.02.2008
(73) Proprietor: Alcon, Inc., 6331 Hünenberg (CH)
(72) Inventor: OWEN, Geoffrey, Robert, Southlake, TX 76092-2871 (US); BROOKS, Amy, C., Burleson, TX 76028 (US); GRAFF, Gustav, Cleburne, TX 76031 (US)
(74) Representative: Teipel, Stephan
(86) International application number: PCT/US2006/017606
(87) International publication number: WO 2006/121963

(56) References cited:
- EP-A- 0 551 626
- EP-A1- 0 550 921
- WO-A-03/074038
- WO-A2-20/04016221
- US-A1- 2003 133 905
- US-A1- 2004 029 771
- US-A1- 2004 106 613

## Description

### BACKGROUND OF THE INVENTION

This invention relates to pharmaceutical compositions for treating ophthalmic disorders. In particular, the present invention relates to topically administrable suspension formulations of nepafenac and other ophthalmic drugs.

Nepafenac is also known as 2-amino-3-benzoylphenylacetamide. The topical use of nepafenac and other amide and ester derivatives of 3-benzoylphenylacetic acid to treat ophthalmic inflammation and pain is disclosed in U.S. Patent No. 5,475,034. According to the '034 patent, compositions containing the 3-benzoylphenylacetic acid derivatives can be formulated into a variety of topically administrable ophthalmic compositions, such as solutions, suspensions, gels, or ointments. The compositions optionally contain preservatives, such as benzalkonium chloride, and thickening agents, such as carbomers, hydroxyethylcellulose or polyvinyl alcohol. The '034 patent, however, does not disclose any formulations of nepafenac or other ophthalmic drugs containing a combination of a poloxamer or meroxapol surfactant and propylene glycol.

Attempts have been made to increase the corneal flux of topically administrable drugs for some time. Many glycols, including propylene glycol are known "penetration enhancers." See, for example, U.S. Patent No. 6,765,001. This patent discloses formulations of corticosteroids for topical application to the skin. The reference formulations contain propylene glycol as a skin penetration enhancer.

Corneal penetration enhancers for topically administrable ophthalmic drugs have also been sought. See, for example, U.S. Patent No. 5,369,095, which discloses the use of dodecyl maltoside as a corneal penetration enhancer. See also, U.S Patent Nos. 6,630,135 and 6,835,392, which in addition to dodecyl maltoside disclose other penetration enhancers for mucosal tissues. These penetration enhancers are intended to increase the corneal penetration of the topically administered drug.

Poloxamer, meroxapol, and poloxamine surfactants are known. They are used in contact lens care solutions and therapeutic ophthalmic compositions including anti-inflammatory compositions. See, for example, U.S. Patent Nos. 6,037,328; 6,544,953; 6,486,215; and 5,631,005.

While poloxamer and meroxapol surfactants (including those commercially available as Pluronic^{®} and Pluronic^{®} R surfactants) and propylene glycol are separately known to be useful in topically administrable ophthalmic compositions, they have not been used in combination with nepafenac and their combined effect on the corneal penetration of sparingly water-soluble ophthalmic drugs has not been disclosed.

### SUMMARY OF THE INVENTION

The compositions of the present invention are aqueous suspension compositions of nepafenac or other ophthalmic drugs that are sparingly soluble in water. The compositions of the present invention comprise a combination of a poloxamer or meroxapol surfactant and a glycol tonicity-adjusting agent. Unlike conventional suspension compositions, the compositions of the present invention do not contain a water-soluble polymeric suspending or viscosifying agent such as a carbopol.

Suspension compositions of sparingly-soluble ophthalmic drugs containing a combination of a poloxamer or meroxapol surfactant and propylene glycol show significantly greater corneal penetration of such drugs than similar compositions that do not contain such a combination of excipients.

### DETAILED DESCRIPTION OF THE INVENTION

Unless indicated otherwise, all ingredient concentrations are presented in units of % weight/volume (% w/v).

As used herein, "sparingly soluble in water" or "sparingly-soluble ophthalmic drug" means a drug that has a solubility limit in water at 25 °C in the range of 0.001 - 0.05 %.

The aqueous compositions of the present invention contain a pharmaceutically effective amount of nepafenac or other sparingly soluble ophthalmic drug. Nepafenac is a known nonsteroidal anti-inflammatory compound. It can be made by known methods. See, for example, U.S. Patent Nos. 5,475,034 and 4,313,949. The nepafenac compositions of the present invention will generally contain 0.01 - 0.3 % (w/v) nepafenac, preferably 0.03 - 0.1 % (w/v) nepafenac.

Particularly with the enhanced corneal penetration of the compositions of the present invention, nepafenac can be used to treat ophthalmic disorders not only of the ocular surface but also of the back of the eye. For example, the topically administrable nepafenac compositions of the present invention may be used to treat ocular surface pain, uveitis, scleritis, episcleritis, keratitis, surgically-induced inflammation, endophthalmitis, iritis, atrophic macular degeneration, retinitis pigmentosa, iatrogenic retinopathy, retinal tears and holes, cystoid macular edema, diabetic macular edema, diabetic retinopathy, sickle cell retinopathy, retinal vein and artery occlusion, optic neuropathy, exudative macular degeneration, neovascular glaucoma, corneal neovascularization, cyclitis, sickle cell retinopathy, and pterygium.

The compositions may contain a sparingly soluble drug compound other than nepafenac. For example, the compositions of the present invention may comprise a sparingly soluble carbonic anhydrase inhibitor, such as brinzolamide; an antifungal agent, such as natamycin; a phosphodiesterase IV inhibitor (PDE-IV or PDE-4) inhibitor, such as roflumilast; a receptor tyrosine kinase inhibitor; a steroid, such as fluorometholone, hydrocortisone, dexamethasone, prednisolone, loteprednol, or medrysone; or a nonsteroidal anti-inflammatory agent that is sparingly soluble in water. All of the foregoing are known compounds and can be made by known methods.

In addition to at least one sparingly soluble ophthalmic drug, the compositions of the present invention comprise a poloxamer nonionic surfactant of formula I or a meroxapol nonionic surfactant of formula II: 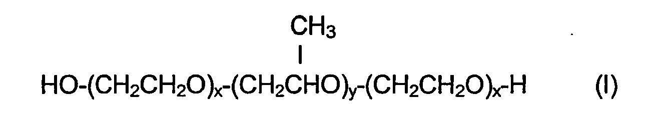 wherein
x is 2 - 125 and y is 5 - 235, provided that 2x is 10 - 80% of 2x + y, and further provided that the number average molecular weight of the poloxamer nonionic surfactant is 1,100 - 14,600; 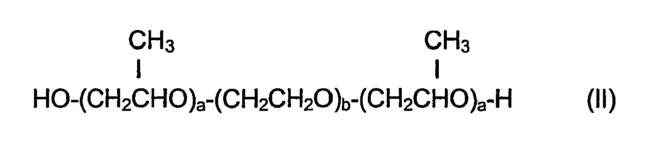
wherein
a is 4 - 60 and b is 4 - 120, provided that b is 10 - 80% of 2a + b, and further provided that the number average molecular weight of the meroxapol nonionic surfactant is 1,900 - 7,000.

Poloxamer and meroxapol nonionic surfactants of formulas I and II above are poly(oxyethylene) and poly(oxypropylene) block copolymers. They are known and are commercially available as Pluronic^{®} and Pluronic^{®} R surfactants from BASF Corporation, Performance Products, Florham Park, New Jersey. Poloxamer and meroxapol are the names adopted for such surfactants by The CTFA International Cosmetic Ingredient Dictionary.

The most preferred poloxamer surfactant is a poloxamer surfactant where x is about 23, y is about 67, and the number average molecular weight of the poloxamer surfactant is about 5,900. This poloxamer surfactant is commercially available as Pluronic^{®} P104.

The compositions of the present invention comprise a total of 0.001 - 0.15 % of a poloxamer surfactant of formula I or a meroxapol surfactant of formula II. Included within the scope of this invention are mixtures of poloxamer surfactants, mixtures of meroxapol surfactants, and mixtures of both poloxamer and meroxapol surfactants. Higher total concentrations of the poloxamer or meroxapol surfactants can reduce the availability of the ophthalmic drug. Preferably, the compositions of the present invention comprise a total of 0.005 - 0.12 % poloxamer or meroxapol surfactant. Most preferably, the compositions of the present invention comprise a total of 0.1 % poloxamer or meroxapol surfactant.

In addition to the ophthalmic drug and the poloxamer or meroxapol surfactant, the compositions of the present invention comprise a glycol tonicity-adjusting agent in a total amount of at least 1 % but less than 4.0 %. The glycol tonicity-adjusting agent is selected from the group consisting of: propylene glycol; glycerol; dipropylene glycol; diethylene glycol; triethylene glycol; 1,3-butylene glycol; 2,3-butylene glycol; 3-methyl-1,3-butylene glycol; diglycerol; erythritol; pentaerythritol; and neopentyl glycol. Included within the scope of this invention are mixtures of glycol tonicity-adjusting agents. Too much glycol tonicity-adjusting agent results in compositions that are uncomfortable when administered because their osmolalities are too high. The compositions of the present invention have osmolalities from 150 - 500 mOsm/Kg. Preferably, the total amount of glycol tonicity-adjusting agent is 2.0 - 3.5 %. Most preferably, the total amount of glycol tonicity-adjusting agent in the compositions of the present invention is 3.0 %. Tonicty-adjusting agents of this type are known and many are commercially available. Preferred glycol tonicity-adjusting agents are propylene glycol, glycerol, and mixtures thereof.

The compositions of the present invention optionally contain metal chloride salts (such as sodium chloride) or non-ionic tonicity adjusting agents (such as mannitol) as additional tonicity-adjusting agents.

The aqueous compositions of the present invention optionally comprise one or more excipients selected from the group consisting of buffering agents, pH-adjusting agents, chelating agents, and preservatives. Buffering agents include phosphate buffers, such as disodium phosphate and monosodium phosphate; borate buffers, such as boric acid and sodium borate; and citrate buffers. The buffering agent is chosen based upon the target pH for the composition, which generally ranges from pH 6.5 - 8.5. The target pH for the composition depends upon the chosen ophthalmic drug. In the case of nepafenac, the desired pH is 7.0 - 8.5, preferably 7.5 - 8.0, and most preferably 7.8. Ophthalmically acceptable pH adjusting agents are known and include, but are not limited to, hydrochloric acid (HCl) and sodium hydroxide (NaOH).

Suitable chelating agents include edetate disodium; edetate trisodium; edetate tetrasodium; and diethyleneamine pentaacetate. Most preferred is edetate disodium. If included, the chelating agent will typically be present in an amount from 0.001 - 0.1 %. In the case of edetate disodium, the chelating agent is preferably present at a concentration of 0.01 %.

Many ophthalmically acceptable preservatives are known and include, but are not limited to, benzalkonium halides and polyquaternium-1. Most preferred preservatives are benzalkonium chloride ("BAC") and polyquaternium-1. In the case of benzalkonium chloride, the preservative is preferably present in an amount from 0.001 - 0.01 %, and most preferably 0.005 %.

The compositions of the present invention optionally comprise a sulfite salt. Examples of sulfite salts include sodium sulfite; potassium sulfite; magnesium sulfite; calcium sulfite; sodium bisulfite; potassium bisulfite; magnesium bisulfite; calcium bisulfite; sodium metabisulfite; potassium metabisulfite; and calcium metabisulfite. If included, the sulfite salt will typically be present in an amount from 0.01 - 1%.

The compositions of the present invention may be prepared by conventional methods of preparing aqueous pharmaceutical suspension compositions, including sizing the drug using known sizing techniques, such as ball-milling. For example, a slurry containing the sparingly soluble drug, a surfactant and sizing beads is tumbled for a time sufficient to obtain drug of desired particle sizes. The sizing beads are then separated from the slurry and the slurry is added to the remaining aqueous ingredients. Preferably, however, the compositions of the present invention are made in a specific manner. According to the preferred method, the drug is first added to a mixture of the poloxamer or meroxapol surfactant and propylene glycol. Preferably, the mixture is warmed (for example, to 50 °C) while the drug is stirred with the mixture to speed up and enhance the dissolution of the drug. After maximizing the dissolution of the drug, the remaining aqueous ingredients (e.g., water, buffering agent, pH-adjusting agent, chelating agent, preservative) are added with vigorous stirring to the dissolved drug. The order of addition to form a mixture of the remaining aqueous ingredients is not critical. This preferred method of preparing the suspension compositions produces a fine suspension of the drug without the need of ball milling to size the drug. In general, target particle sizes for the suspension compositions of the present invention range from 0.1 - 100 µm, and preferably range from 0.5 - 50 µm.

The following examples are intended to illustrate, but not limit, the present invention.

### Example 1

The formulation shown below is representative of the compositions of the present invention.

| | **1** | **1A** |
|---|---|---|
| **INGREDIENT** | % (w/v) | % (w/v) |
| Nepafenac | 0.1 | 0.1 |
| Poloxamer (Pluronic^{®} P104) | 0.1 | 0.1 |
| Propylene Glycol | 3.0 | 3.0 |
| Edetate Disodium | 0.01 | 0.01 |
| Benzalkonium Chloride | 0.005 | 0.005 |
| Boric Acid | 0.06 | 0.06 |
| Sodium Borate | 0.02 | 0.02 |
| Sodium Sulfite | --- | 0.09 |
| NaOH/HCl | q.s. pH 7.5 - 8.0 | q.s. pH 7.5 - 8.0 |
| Purified Water | q.s. 100 | q.s. 100 |

### Example 2

The formulation shown below is representative of the compositions of the present invention.

| | **2** |
|---|---|
| **INGREDIENT** | % (w/v) |
| PDE-IV Inhibitor | 1.0 |
| Poloxamer (Pluronic^{®} P104) | 0.1 |
| Propylene Glycol | 3.0 |
| Edetate Disodium | 0.01 |
| Benzalkonium Chloride | 0.005 |
| Disodium Phosphate | 0.1 - 0.2 |
| NaOH/HCl | q.s. pH 7.2 - 8.0 |
| Purified Water | q.s. 100 |

### Example 3

The formulations shown in Table 1 were prepared and evaluated in an *ex vivo* cornea! permeation model. The corneal penetration results are also shown in Table 1. Formulations A - C were prepared by ball-milling nepafenac in a slurry containing tyloxapol and/or polysorbate 80 for approximately 18 hours. Formulation AA was prepared by dissolving the nepafenac in a mixture of Pluronic^{®} P-104 and propylene glycol, then adding the remaining ingredients. The ex *vivo* corneal penetration rabbit model is briefly described below:
Rabbits were sacrificed by first anaesthetizing with ketamine (30mg/Kg) and xylazine (6mg/Kg) followed by an injection of an overdose of SLEEPAWAY® (sodium pentobarbital, 1ml of a 26% solution) into the marginal ear vein. The intact eyes, along with the lids and conjunctival sacs were then enucleated and immediately stored in about 70 ml of fresh BSS PLUS@ irrigation solution saturated with O₂/CO₂ (95:5). Within one hour, the enucleated rabbit eyes were mounted in the modified perfusion chambers as described by Schoenwald, et al., "Corneal Penetration Behavior of β-Blocking Agents I: Physiochemical Factors," Journal of Pharmaceutical Sciences, 72(11) ( November 1983). After mounting in the chambers, 7.5 mls of BSS PLUS@ was placed in the receiving side of the chamber with stirring and bubbling and immediately capped to prevent contamination. Then, 7 mls of each test formulation was dosed on the donor side of the chamber for 5 minutes with stirring and bubbling. Afterwards, the donor chamber was emptied with suction and filled with 7mls of BSS PLUS@ for approximately 15 seconds. This suction and rinsing with BSS PLUS@ was repeated 7 times, and on the 8th fill, the BSS PLUS@ was left in the donor chamber. Samples were withdrawn from the receiving chamber every 30 minutes over a five hour period, and the levels of test drug were determined using HPLC. The rate of drug accumulation in the receiver compartment and 5 hour accumulations were then calculated from graphs of the data.

The solubility of the test drug was determined using HPLC analysis after filtering the test formulation through a 0.25 micron screen.

**TABLE 1**

| | Formulation (% w/v) | | | |
|---|---|---|---|---|
| **Ingredient** | **A** | **B** | **C** | **AA** |
| Nepafenac | 0.1 | 0.1 | 0.1 | 0.1 |
| Carbopol 974P | 0.5 | 0.5 | --- | --- |
| Sodium Chloride | 0.4 | 0.4 | 0.28 | --- |
| Mannitol | 2.4 | 2.4 | --- | --- |
| Tyloxapol | 0.01 | 0.01 | --- | --- |
| Disodium Phosphate | --- | --- | 0.18 | --- |
| Boric Acid | --- | --- | --- | 0.07 |
| Pluronic® P-104 | --- | --- | --- | 0.1 |
| Propylene Glycol | --- | --- | --- | 3 |
| Polyethylene Glycol | --- | --- | 5 | --- |
| Polysorbate 80 | --- | --- | 0.5 | --- |
| Hydroxypropylmethyl cellulose (HPMC 2910) | --- | --- | 0.5 | --- |
| Dodecyl Maltoside | --- | 0.05 | --- | --- |
| Edetate Disodium | 0.01 | 0.01 | --- | 0.01 |
| Benzalkonium Chloride | 0.005 | 0.005 | --- | 0.005 |
| NaOH/HCl q.s. to pH | 7.5 | 7.5 | 7.5 | 7.8 |
| Osmolality (mOsm) | --- | 296 | 330 | 371 |
| Solubility (ppm) | 26 | 16 | 49 | 21 |
| Rate of Accumulation (µg/min) | 0.0126 | 0.011 | 0.0108 | 0.049 |
| Standard Deviation | 0.0007 | 0.002 | 0.0001 | 0.006 |
| 5 hour accumulation (µg) | 4.2 | 3.8 | 3.5 | 13.5 |
| Standard Deviation | 0.2 | 0.6 | 0.1 | 1.4 |

Formulation B is the same as Formulation A with the known penetration enhancer dodecyl maltoside ("DDM") added. The results show that the penetration of B is slightly inferior to A, showing that DDM is not an effective penetration enhancer in the tested formulation.

Formulation C is a viscous formulation containing polyethylene glycol (5%). The solubility of nepafenac is almost doubled compared to Formulation A, but the penetration results are inferior to A.

Formulation AA is a formulation according to the present invention. It contains a combination of a poloxamer surfactant and propylene glycol. The penetration results are superior to A.

### Example 4

The formulations shown in Table 2 were prepared and evaluated in the *ex vivo* corneal penetration model described above. The corneal penetration results are also shown in Table 2. All Formulations were prepared in the same manner as Formulation AA.

**TABLE 2**

| | **Formulation (% w/v)** | | | | | | | | | |
|---|---|---|---|---|---|---|---|---|---|---|
| **Ingredient** | **D** | **BB** | **CC** | **AA** | **DD** | **EE** | **FF** | **GG** | **HH** | **II** |
| **Nepafenac** | **0.1** | **0.1** | **0.1** | **0.1** | **0.1** | **0.1** | **0.1** | **0.1** | **0.1** | **0.1** |
| **Disodium Phosphate** | **0.16** | **---** | **---** | **---** | **---** | **---** | **---** | **---** | **---** | **---** |
| **Boric Acid** | **---** | **0.07** | **0.07** | **0.07** | **0.07** | **0.07** | **0.07** | **0.07** | **0.07** | **0.07** |
| **Pluronic® P-104** | **---** | **0.005** | **0.05** | **0.1 -** | **0.2** | **0.5** | **1** | **1.5** | **2** | **3** |
| **Propylene Glycol** | **3** | **3** | **3** | **3** | **3** | **3** | **3** | **3** | **3** | **3** |
| **Edetate Disodium** | **0.01** | **0.01** | **0.01** | **0.01** | **0.01** | **0.01** | **0.01** | **0.01** | **0.001** | **0.001** |
| **Benzalkonium Chloride** | **0.005** | **0.005** | **0.005** | **0.005** | **0.005** | **0.005** | **0.005** | **0.005** | **0.005** | **0.005** |
| **NaOH/HCl q.s. to pH** | **7.8** | **7.87** | **7.88** | **7.86** | **7.81** | **7.82** | **7.84** | **7.82** | **7.84** | **7.86** |
| **Osmolality (mOsm)** | **439** | **415** | **376** | **371** | **371** | **359** | **370** | **380** | **391** | **403** |
| **Solubility (ppm)** | **15** | **21** | **19** | **21** | **24** | **33** | **26** | **40** | **52** | **70** |

| **Ex Vivo Corneal Penetration Results** | | | | | | | | | | |
|---|---|---|---|---|---|---|---|---|---|---|
| **Rate of Accumulation (µg/mn)** | **0.035** | **0.053** | **0.053** | **0.049** | **0.031** | **0.038** | **0.029** | **0.025** | **0.037** | **0.035** |
| **Standard Deviation** | **0.004** | **0.009** | **0.004** | **0.006** | **0.005** | **0.001** | **0.002** | **0.002** | **0.001** | **0.01** |
| **5 hour accumulation (µg)** | **9.6** | **14.9** | **14.6** | **13.5** | **8.6** | **10.6** | **8.1** | **7.0** | **10.1** | **9.6** |
| **Standard Deviation** | **1.0** | **2.1** | **0.9** | **1.4** | **1.5** | **0.2** | **0.3** | **0.6** | **0.2** | **2.8** |

Each of the formulations shown in Table 2 contains 3 % propylene glycol. The amount of poloxamer surfactant (Pluronic^{®} P-104) is varied from 0 % (Formulation D) to 3 % (Formulation II). The results show that over this range, the solubility of nepafenac increases from 15 ppm to 70 ppm. The drug penetration data, however, show that corneal drug penetration increases with increasing poloxamer concentration up to a poloxamer concentration of 0.1 %, then corneal penetration decreases with increasing poloxamer concentration.

### Example 5

The formulations shown in Table 3 were prepared and evaluated in the ex *vivo* corneal penetration model described above. The corneal penetration results are also shown in Table 3. Formulation E was prepared in the same manner as Formulation A. Formulation JJ was prepared in the same manner as Formulation AA.

**TABLE 3**

| | **Formulation (% w/v)** | |
|---|---|---|
| **Ingredient** | **E** | **JJ** |
| Brinzolamide | 1 | 1 |
| Carbomer 974P | 0.4 | --- |
| Boric Acid | --- | 0.07 |
| Mannitol | 3.3 | --- |
| Tyloxapol | 0.025 | --- |
| Sodium Chloride | 0.25 | --- |
| Pluronic® P-104 | --- | 0.1 |
| Propylene Glycol | --- | 3 |
| Edetate Disodium | 0.01 | 0.01 |
| Benzalkonium Chloride | 0.01 | 0.005 |
| NaOH/HCl q.s to pH | 7.5 | 7.87 |
| Osmolality (mOsm) | 300 | 390 |
| Solubility (ppm) | 425 | 529 |

| **Ex Vivo Corneal Penetration Results** | | |
|---|---|---|
| Rate of Accumulation (µg/min) | 0.0071 | 0.20 |
| Standard Deviation | 0.0001 | 0.05 |
| 5 hour Accumulation | 2.8 | 50 |
| Standard Deviation | 0.3 | 9 |

The penetration results shown in Table 3 demonstrate that the compositions of the present invention possess superior corneal penetration when the drug is not nepafenac but is another sparingly soluble ophthalmic drug. In this case, the sparingly soluble ophthalmic drug is the carbonic anhydrase inhibitor known as brinzolamide.

### Example 6

The formulations shown in Table 4 were prepared and evaluated in the *ex vivo* corneal penetration model described above. The corneal penetration results are also shown in Table 4. Formulation F was prepared in the same manner as Formulation A. Formulation KK was prepared in the same manner as Formulation AA.

**TABLE 4**

| | **Formulation** | **(% w/v)** |
|---|---|---|
| **Ingredient** | **F** | **KK** |
| Dexamethasone | 0.1 | 0.1 |
| Boric Acid | --- | 0.07 |
| Polysorbate 80 | 0.05 | --- |
| Dibasic Sodium Phosphate | 0.2 | --- |
| Hydroxypropyl Methylcellulose | 0.5 | --- |
| Pluronic® P-104 | --- | 0.1 |
| Propylene Glycol | --- | 3 |
| Edetate Disodium | 0.01 | 0.01 |
| Benzalkonium Chloride | 0.01 | 0.005 |
| NaOH/HCl q.s to pH | 5.4 | 7.89 |
| Osmolality (mOsm) | 300 | 422 |
| Solubility (ppm) | 85 | 92 |

| **Ex Vivo Corneal Penetration Results** | | |
|---|---|---|
| Rate of Accumulation (µg/min) | 0.0015 | 0.019 |
| Standard Deviation | 0.0003 | 0.004 |
| 5 hour Accumulation | 0.59 | 5.0 |
| Standard Deviation | 0.1 | 1.5 |

The penetration results shown in Table 4 demonstrate that the compositions of the present invention possess superior corneal penetration when the drug is not nepafenac but is another sparingly soluble ophthalmic drug. In this case, the sparingly soluble ophthalmic drug is dexamethasone.

## Claims

1. A topically administrable aqueous ophthalmic suspension composition comprising
a) an ophthalmic drug having a solubility in water at 25 °C from 0.001 - 0.05 % (w/v);
b) a poloxamer or meroxapol nonionic surfactant in an amount of 0.001 - 0.15 % (w/v);
c) a glycol tonicity-adjusting agent selected from the group consisting of: propylene glycol; glycerol; dipropylene glycol; diethylene glycol; triethylene glycol; 1,3-butylene glycol; 2,3-butylene glycol; 3-methyl-1,3-butylene glycol; diglycerol; erythritol; pentaerythritol; and neopentyl glycol, in an amount of at least 1.0 % (w/v) but less than 4.0 % (w/v); and
d) water;
wherein the composition has an osmolality from 150 - 500 mOsm/Kg and wherein the poloxamer nonionic surfactant has the formula 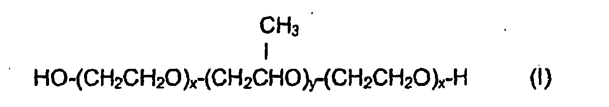 wherein
x is 2 -125 and y is 5 - 235, provided that 2x is 10 - 80% of 2x + y, and further provided that the number average molecular weight of the poloxamer nonionic surfactant is 1,100 - 14,600;
and the meroxapol nonionic surfactant has the formula 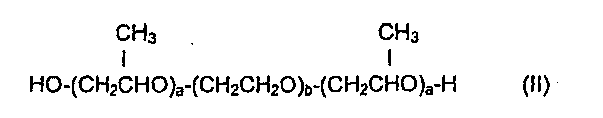 wherein
a is 4 - 60 and b is 4 - 120, provided that b is 10 - 80% of 2a + b, and further provided that the number average molecular weight of the meroxapol nonionic surfactant is 1,900 - 7,000.

2. The composition of Claim 1 wherein the ophthalmic drug is selected from the group consisting of nonsteroidal anti-inflammatory compounds; carbonic anhydrase inhibitors; antifungal agents; phosphodiesterase IV inhibitors; receptor tyrosine kinase inhibitors; and steroids.

3. The composition of Claim 2 wherein the ophthalmic drug is selected from the group consisting of nepafenac; brinzolamide; natamycin; roflumilast; fluorometholone; hydrocortisone; dexamethasone; prednisolone; loteprednol; and medrysone.

4. The composition of Claim 1 wherein the ophthalmic drug is nepafenac.

5. The composition of Claim 1 wherein the poloxamer or meroxapol nonionic surfactant is a poloxamer nonionic surfactant of formula (I).

6. The composition of Claim 1 wherein the poloxamer or meroxapol nonionic surfactant is a meroxapol nonionic surfactant of formula (II).

7. The composition of Claim 1 wherein the poloxamer or meroxapol nonionic surfactant is present in an amount from 0.005 - 0.12 % (w/v).

8. The composition of Claim 7 wherein the poloxamer or meroxapol nonionic surfactant is present in an amount of 0.1 % (w/v).

9. The composition of Claim 1 wherein the glycol tonicity-adjusting agent is selected from the group consisting of: propylene glycol; glycerol; and mixtures thereof.

10. The composition of Claim 1 wherein the glycol tonicity-adjusting agent is present in an amount from 2.0 - 3.5 % (w/v).

11. The composition of Claim 10 wherein the glycol tonicity-adjusting agent is present in an amount of 3.0 % (w/v).

12. The composition of Claim 1 wherein the composition further comprises a tonicity-adjusting agent selected from the group consisting of metal chloride salts and non-ionic tonicity adjusting agents.

13. The composition of Claim 1 wherein the composition further comprises an excipient selected from the group consisting of buffering agents; pH-adjusting agents; chelating agents; and preservatives.

14. The composition of Claim 1 wherein the composition lacks a polymeric suspending agent.

15. A topically administrable aqueous ophthalmic suspension composition of claim 1 comprising
a) 0.01 - 0.3 % (w/v) nepafenac;
b) 0.001 - 0.15 % (w/v) poloxamer or meroxapol nonionic surfactant;
c) 2.0 - 3.5 % (w/v) glycol tonicity-adjusting agent is selected from the group consisting of: propylene glycol; glycerol; and mixtures thereof;
d) 0.001 - 0.1 % (w/v) edetate disodium;
e) 0.001 - 0.01 % (w/v) of an ophthalmically acceptable preservative; and
f) water;
wherein the composition has a pH from 7.5 - 8.0 and an osmolality from 250 - 500 mOsm/Kg, and wherein the poloxamer nonionic surfactant has the formula 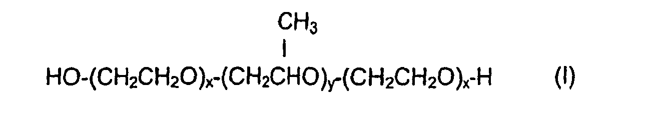 wherein
x is 2 -125 and y is 5 - 235, provided that 2x is 10 - 80% of 2x + y, and further provided that the number average molecular weight of the poloxamer nonionic surfactant is 1,100 - 14,600;
and the meroxapol nonionic surfactant has the formula 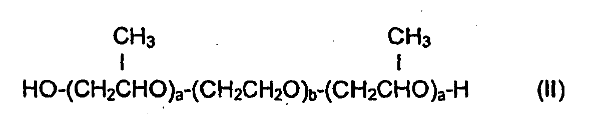 wherein
a is 4 - 60 and b is 4 - 120, provided that b is 10 - 80% of 2a + b, and further provided that the number average molecular weight of the meroxapol nonionic surfactant is 1,900 - 7,000.

16. The composition of Claim 15 wherein the composition further comprises a sulfite salt selected from the group consisting of sodium sulfite; potassium sulfite; magnesium sulfite; calcium sulfite; sodium bisulfite; potassium bisulfite; magnesium bisulfite; calcium bisulfite; sodium metabisulfite; potassium metabisulfite; and calcium metabisulfite.

17. Use of an aqueous suspension composition comprising
a) a pharmaceutically effective amount of nepafenac;
b) a poloxamer or meroxapol nonionic surfactant in an amount of 0.001 - 0.15 % (w/v);
c) a glycol tonicity-adjusting agent in an amount of at least 1.0 % (w/v) but less than 4.0 % (w/v); and
d) water;
wherein the composition has an osmolality from 150 - 500 mOsm/Kg,
the poloxamer nonionic surfactant has the formula 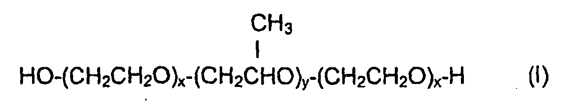 wherein
x is 2 - 125 and y is 5 - 235, provided that 2x is 10 - 80% of 2x + y, and further provided that the number average molecular weight of the poloxamer nonionic surfactant is 1,100 - 14,600;
and the meroxapol nonionic surfactant has the formula 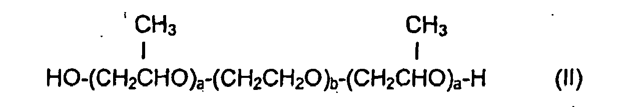 wherein
a is 4 - 60 and b is 4 - 120, provided that b is 10 - 80% of 2a + b, and further provided that the number average molecular weight of the meroxapol nonionic surfactant is 1,900 - 7,000,
the glycol tonicity-adjusting agent is selected from the group consisting of: propylene glycol; glycerol; dipropylene glycol; diethylene glycol; triethylene glycol; 1,3-butylene glycol; 2,3-butylene glycol; 3-methyl-1,3-butylene glycol; diglycerol; erythritol; pentaerythritol; and neopentyl glycol,
for the manufacture of a topical medicament for the treatment of an ophthalmic disorder, wherein the ophthalmic disorder is selected from the group consisting of ocular surface pain; uveitis; scleritis; episcleritis; keratitis; surgically-induced inflammation; endophthalmitis; iritis; atrophic macular degeneration; retinitis pigmentosa; iatrogenic retinopathy; retinal tears and holes; cystoid macular edema; diabetic macular edema; diabetic retinopathy; sickle cell retinopathy; retinal vein and artery occlusion; optic neuropathy; exudative macular degeneration; neovascular glaucoma; corneal neovascularization; cyclitis; sickle cell retinopathy; and pterygium.

18. The use of Claim 17 wherein the composition comprises
a) 0.01 - 0.3 % (w/v) nepafenac;
b) 0.001 - 0.15 % (w/v) of the poloxamer or meroxapol nonionic surfactant;
c) 2.0 - 3.5 % (w/v) glycol tonicity-adjusting agent is selected from the group consisting of: propylene glycol; glycerol; and mixtures thereof;
d) 0.001 - 0.1 % (w/v) edetate disodium;
e) 0.001 - 0.01 % (w/v) of an ophthalmically acceptable preservative; and
f) water;
wherein the composition has a pH from 7.5 - 8.0.

## Patentansprüche

1. Topisch verabreichbare wässerige ophthalmische Suspensionszusammensetzung, umfassend
a) ein ophthalmisches Arzneimittel mit einer Löslichkeit in Wasser bei 25 °C von 0,001 - 0,05 % (Gew./V.);
b) ein nicht-ionisches oberflächenaktives Mittel auf der Basis von Poloxamer oder Meroxapol in einer Menge von 0,001 - 0,15 % (Gew./V.);
c) ein Tonizitätseinstellmittel auf der Basis von Glycol, ausgewählt aus der Gruppe, bestehend aus Propylenglycol; Glycerol; Dipropylenglycol; Diethylenglycol; Triethylenglycol; 1,3-Butylenglycol; 2,3-Butylenglycol; 3-Methyl-1,3-butylenglycol; Diglycerol; Erythritol; Pentaerythritol und Neopentylglycol, in einer Menge von mindestens 1,0 % (Gew./V.), aber weniger als 4,0 % (Gew./V.); und
d) Wasser;
wobei die Zusammensetzung eine Osmolalität von 150 - 500 mOsm/kg hat und wobei das nicht-ionische oberflächenaktive Mittel auf der Basis von Poloxamer die Formel 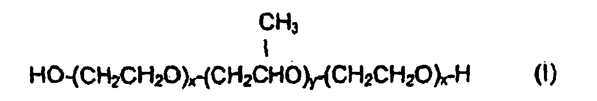 aufweist,
worin
x 2 - 125 ist und y 5 - 235 ist, vorausgesetzt, dass 2x 10 - 80 % von 2x + y ist, und ferner vorausgesetzt, dass das zahlenmittlere Molekulargewicht des nicht-ionischen oberflächenaktiven Mittels auf der Basis von Poloxamer 1.100 - 14.600 beträgt;
und das nicht-ionische oberflächenaktive Mittel auf der Basis von Meroxapol die Formel 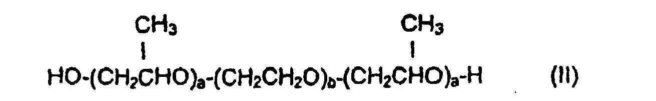 aufweist,
worin
a 4 - 60 ist und b 4 - 120 ist, vorausgesetzt, dass b 10 - 80 % von 2a + b ist, und ferner vorausgesetzt, dass das zahlenmittlere Molekulargewicht des nicht-ionischen oberflächenaktiven Mittels auf der Basis von Meroxapol 1.900 - 7.000 beträgt.

2. Zusammensetzung nach Anspruch 1, wobei das ophthalmische Arzneimittel aus der Gruppe ausgewählt ist, bestehend aus nicht-steroidalen entzündungshemmenden Verbindungen; Carboanhydraseinhibitoren; Antimykotika; Phosphodiesterase IV-Inhibitoren; Rezeptortyrosinkinaseinhibitoren und Steroiden.

3. Zusammensetzung nach Anspruch 2, wobei das ophthalmische Arzneimittel aus der Gruppe ausgewählt ist, bestehend aus Nepafenac; Brinzolamid; Natamycin; Roflumilast; Fluorometholon; Hydrocortison; Dexamethason; Prednisolon; Loteprednol und Medryson.

4. Zusammensetzung nach Anspruch 1, wobei das ophthalmische Arzneimittel Nepafenac ist.

5. Zusammensetzung nach Anspruch 1, wobei das nicht-ionische oberflächenaktive Mittel auf der Basis von Poloxamer oder Meroxapol ein nicht-ionisches oberflächenaktives Mittel auf das Basis von Poloxamer der Formel (I) ist.

6. Zusammensetzung nach Anspruch 1, wobei das nicht-ionische oberflächenaktive Mittel auf der Basis von Poloxamer oder Meroxapol ein nicht-ionisches oberflächenaktives Mittel auf das Basis von Meroxapol der Formel (II) ist.

7. Zusammensetzung nach Anspruch 1, wobei das nicht-ionische oberflächenaktive Mittel auf der Basis von Poloxamer oder Meroxapol in einer Menge von 0,005 - 0,12 % (Gew./V.) vorliegt.

8. Zusammensetzung nach Anspruch 7, wobei das nicht-ionische oberflächenaktive Mittel auf der Basis von Poloxamer oder Meroxapol in einer Menge von 0,1 % (Gew./V.) vorliegt.

9. Zusammensetzung nach Anspruch 1, wobei das Tonizitätseinstellmittel auf der Basis von Glycol aus der Gruppe ausgewählt ist, bestehend aus: Propylenglycol; Glycerol und Gemischen davon.

10. Zusammensetzung nach Anspruch 1, wobei das Tonizitätseinstellmittel auf der Basis von Glycol in einer Menge von 2,0 - 3,5 % (Gew./V.) vorliegt.

11. Zusammensetzung nach Anspruch 10, wobei das Tonizitätseinstellmittel auf der Basis von Glycol in einer Menge von 3,0 % (Gew./V.) vorliegt.

12. Zusammensetzung nach Anspruch 1, wobei die Zusammensetzung ferner ein Tonizitätseinstellmittel, ausgewählt aus der Gruppe, bestehend aus Metallchloridsalzen und nicht-ionischen Tonizitätseinstellmitteln, umfasst.

13. Zusammensetzung nach Anspruch 1, wobei die Zusammensetzung ferner einen Hilfsstoff, ausgewählt aus der Gruppe, bestehend aus Puffern, pH-Einstellmitteln, Chelatbildnern und Konservierungsmitteln, umfasst.

14. Zusammensetzung nach Anspruch 1, wobei die Zusammensetzung kein polymeres Suspendiermittel aufweist.

15. Topisch verabreichbare wässerige ophthalmische Suspensionszusammensetzung von Anspruch 1, umfassend
a) 0,01 - 0,3 % (Gew./V.) Nepafenac;
b) 0,001 - 0,15 % (Gew./V.) nicht-ionisches oberflächenaktives Mittel auf der Basis von Poloxamer oder Meroxapol;
c) 2,0 - 3,5 % (Gew./V.) Tonizitätseinstellmittel auf der Basis von Glycol, ausgewählt aus der Gruppe, bestehend aus: Propylenglycol; Glycerol und Gemischen davon;
d) 0,001 - 0,1 % (Gew./V.) Edetatdinatrium;
e) 0,001 - 0,01 % (Gew./V.) eines ophthalmisch akzeptablen Konservierungsmittels und
f) Wasser;
wobei die Zusammensetzung einen pH von 7,5 - 8,0 und eine Osmolalität von 250 - 500 mOsm/kg hat, und wobei das nicht-ionische oberflächenaktive Mittel auf der Basis von Poloxamer die Formel 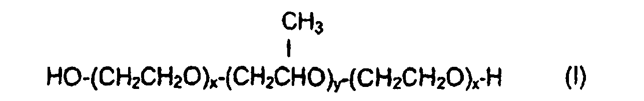 aufweist,
worin
x 2 - 125 ist und y 5 - 235 ist, vorausgesetzt, dass 2x 10 - 80 % von 2x + y ist, und ferner vorausgesetzt, dass das zahlenmittlere Molekulargewicht des nicht-ionischen oberflächenaktiven Mittels auf der Basis von Poloxamer 1.100 - 14.600 beträgt;
und das nicht-ionische oberflächenaktive Mittel auf der Basis von Meroxapol die Formel 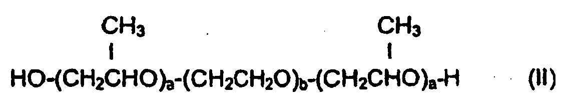 aufweist,
worin
a 4 - 60 ist und b 4 - 120 ist, vorausgesetzt, dass b 10 - 80 % von 2a + b ist, und ferner vorausgesetzt, dass das zahlenmittlere Molekulargewicht des nicht-ionischen oberflächenaktiven Mittels auf der Basis von Meroxapol 1.900 - 7.000 beträgt.

16. Zusammensetzung nach Anspruch 15, wobei die Zusammensetzung ferner ein Sulfitsalz, ausgewählt aus der Gruppe, bestehend aus Natriumsulfit; Kaliumsulfit; Magnesiumsulfit; Calciumsulfit; Natriumbisulfit; Kaliumbisulfit; Magnesiumbisulfit; Calciumbisulfit; Natriummetabisulfit; Kaliummetabisulfit und Calciummetabisulfit, umfasst.

17. Verwendung einer wässerigen Suspensionszusammensetzung, umfassend
a) eine pharmazeutisch wirksame Menge Nepafenac;
b) ein nicht-ionisches oberflächenaktives Mittel auf der Basis von Poloxamer oder Meroxapol in einer Menge von 0,001 - 0,15 % (Gew./V.);
c) ein Tonizitätseinstellmittel auf der Basis von Glycol in einer Menge von mindestens 1,0 % (Gew./V.), aber weniger als 4,0 % (Gew./V.) und
d) Wasser;
wobei die Zusammensetzung eine Osmolalität von 150 - 500 mOsm/kg hat,
das nicht-ionische oberflächenaktive Mittel auf der Basis von Poloxamer die Formel 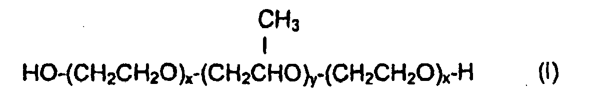 aufweist,
worin
x 2 - 125 ist und y 5 - 235 ist, vorausgesetzt, dass 2x 10 - 80 % von 2x + y ist, und ferner vorausgesetzt, dass das zahlenmittlere Molekulargewicht des nicht-ionischen oberflächenaktiven Mittels auf der Basis von Poloxamer 1.100 - 14.600 beträgt;
und das nicht-ionische oberflächenaktive Mittel auf der Basis von Meroxapol die Formel 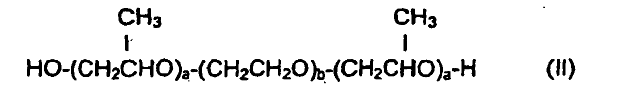 aufweist,
worin
a 4 - 60 ist und b 4 - 120 ist, vorausgesetzt, dass b 10 - 80 % von 2a + b ist, und ferner vorausgesetzt, dass das zahlenmittlere Molekulargewicht des nicht-ionischen oberflächenaktiven Mittels auf der Basis von Meroxapol 1.900 - 7.000 beträgt,
das Tonizitätseinstellmittel auf der Basis von Glycol aus der Gruppe ausgewählt ist, bestehend aus: Propylenglycol; Glycerol; Dipropylenglycol; Diethylenglycol; Triethylenglycol; 1,3-Butylenglycol; 2,3-Butylenglycol; 3-Methyl-1,3-butylenglycol; Diglycerol; Erythritol; Pentaerythritol und Neopentylglycol,
zur Herstellung eines topischen Medikaments zur Behandlung einer ophthalmischen Störung, wobei die ophthalmische Störung aus der Gruppe ausgewählt ist, bestehend aus Augenoberflächenschmerz, Uveitis, Skleritis, Episkleritis, Keratitis, operativ-induzierter Entzündung, Endophthalmitis, Iritis, atropher Makuladegeneration, Retinitis pigmentosa, iatrogener Retinopathie, Netzhautrissen und -löchern, zystoidem Makulaödem, diabetischem Makulaödem, diabetischer Retinopathie, Sichelzellenretinopathie, Netzhautvenen- und -arterienverschluss, optischer Neuropathie, exsudativer Makuladegeneration, neovaskulärem Glaukom, Hornhautneovaskularisation, Ziliarkörperentzündung, Sichelzellenretinopathie und Pterygium.

18. Verwendung nach Anspruch 17, wobei die Zusammensetzung
a) 0,01 - 0,3 % (Gew./V.) Nepafenac;
b) 0,001 - 0,15 % (Gew./V.) des nicht-ionischen oberflächenaktiven Mittels auf der Basis von Poloxamer oder Meroxapol;
c) 2,0 - 3,5 % (Gew./V.) Tonizitätseinstellmittel auf der Basis von Glycol, ausgewählt aus der Gruppe, bestehend aus: Propylenglycol; Glycerol und Gemischen davon;
d) 0,001 - 0,1 % (Gew./V.) Edetatdinatrium;
e) 0,001 - 0,01 % (Gew./V.) eines ophthalmisch akzeptablen Konservierungsmittels und
f) Wasser umfasst;
wobei die Zusammensetzung einen pH von 7,5 - 8,0 hat.

## Revendications

1. Composition de suspension ophtalmique aqueuse pouvant être administrée topiquement comprenant:
a) un médicament ophtalmique d'une solubilité à l'eau à 25°C allant de 0,001 à 0,05% (pds/v);
b) un surfactant non ionique poloxamère ou péroxapol dans une quantité allant de 0,001 à 0,15% (pds/v);
c) un agent réglant la tonicité du glycol sélectionné parmi le groupe composé des suivants: propylène glycol, glycérol, dipropylène glycol, diéthylène glycol, triéthylène glycol, 1,3-butylène glycol, 2,3-butylène glycol, 3-méthyl-1,3-butylène glycol, diglycérol, étythritol, pentaérythritol et néopentyl glycol, dans une quantité d'au moins 1,0% (pds/v) mais de moins que 4,0% (pds/v), et
d) de l'eau;
dans laquelle la composition a une osmolalité de 150 à 500 mOsm/kg et dans laquelle le surfactant non ionique poloxamère présente la formule suivante 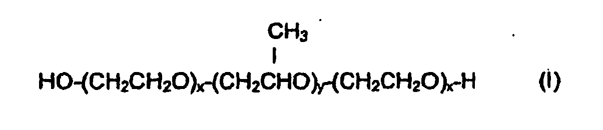 dans laquelle
x est 2 à 125 et y est de 5 à 235, pourvu que 2x soit de 10 à 80% de 2x + y et toujours pourvu que le poids moléculaire moyen en nombre du surfactant non ionique poloxamère aille de 1.00 à 14.600,
et le surfactant non ionique méroxapol a la formule suivante 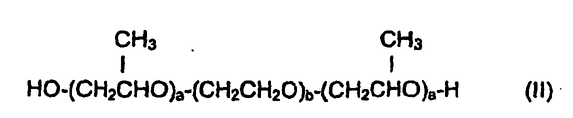 dans laquelle
a est de 4 à 60 et b est de 4 à 120, pourvu que b soit de 10 à 80% de 2a + b et toujours pourvu que le poids moléculaire moyen en nombre du surfactant non ionique méroxapol aille de 1.900 à 7.000.

2. Composition selon la revendication 1, dans laquelle le médicament ophtalmique est sélectionné parmi le groupe constitué des composés anti-inflammatoires non stéroïdaux suivants: inhibiteurs de l'anhydrase carbonique, agents antifongiques, inhibiteurs de la phosphodiestérase IV, inhibiteurs du récepteur tyrosine kinase et des stéroïdes.

3. Composition selon la revendication 2, dans laquelle le médicament ophtalmique est sélectionné parmi le groupe constitué des suivants: népafénac, brinzolamide, natamycine, roflumilast, fluorométholone, hydrocortisone, dexaméthasone, prédnisolone, lotéprédnol et médrysone.

4. Composition selon la revendication 1, dans laquelle le médicament ophtalmique est le népafénac.

5. Composition selon la revendication 1, dans laquelle le surfactant non ionique poloxamère ou méroxapol est un surfactant non ionique poloxamère de formule (I).

6. Composition selon la revendication 1, dans laquelle le surfactant non ionique poloxamère ou méroxapol est un surfactant non ionique méroxapol de formule (II).

7. Composition selon la revendication 1, dans laquelle le surfactant non ionique poloxamère ou méroxapol est présent dans une quantité de 0,005 à 0,12% (pds/v).

8. Composition selon la revendication 7, dans laquelle le surfactant non ionique poloxamère ou méroxapol est présent dans une quantité de 0,1% (pds/v).

9. Composition selon la revendication 1, dans laquelle l'agent de réglage de tonicité du glycol est sélectionné parmi les groupe composé des suivants: propylène glycol, glycérol et des mélanges de ceux-ci.

10. Composition selon la revendication 1, dans laquelle l'agent de réglage de tonicité du glycol est présent dans une quantité de 2,0 à 3,5% (pds/v).

11. Composition selon la revendication 10, dans laquelle l'agent de réglage de tonicité du glycol est présent dans une quantité de 3,0% (pds/v).

12. Composition selon la revendication 1, dans laquelle la composition comprend en outre un agent de réglage de tonicité sélectionné parmi le groupe composé des sels de chlorure de métal et d'agents de réglage de tonicité non ioniques.

13. Composition selon la revendication 1, dans laquelle la composition comprend en outre un excipient sélectionné parmi le groupe composé des agents de tampon, des agents de réglage du pH, des agents de chélation et des conservateurs.

14. Composition selon la revendication 1, dans laquelle composition il manque un agent de mise en suspension polymère.

15. Composition de suspension ophtalmique aqueuse pouvant être administrée topiquement selon la revendication 1 comprenant:
a) de 0,01 à 0,3% (pds/v) de népafénac;
b) de 0,001 à 0,15% (pds/v) de surfactant non ionique poloxamère ou méroxapol;
c) de 2,0 à 3,5% (pds/v) d'un agent de réglage de tonicité du glycol qui est sélectionné parmi le groupe composé des suivants: propylène glycol, glycérol et des mélanges de ceux-ci;
d) de 0,001 à 0,1% (pds/v) d'édétate disodium;
e) de 0,001 à 0,01% (pds/v) d'un conservateur acceptable sur le plan ophtalmique, et
f) de l'eau
dans laquelle la composition a un pH compris entre 7,5 et 8,0 et une osmolalité de 250 à 500 mOsm/kg, et dans laquelle le surfactant non ionique poloxamère présente la formule suivante: 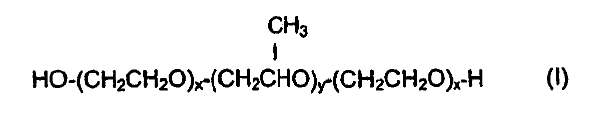 dans laquelle
x est 2 à 125 et y est 5 à 235, pourvu que 2x soit 10 à 80% de 2x + y et toujours pourvu que le poids moléculaire moyen en nombre du surfactant non ionique poloxamère soit de 1.100 à 14.600;
et le surfactant non ionique méroxapol présente la formule suivante: 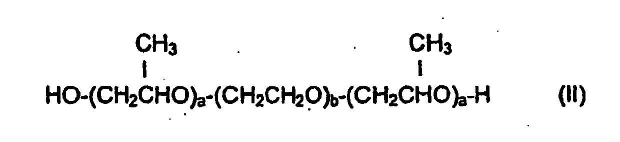 dans laquelle
a est 4 à 60 et b est 4 à 120, pourvu que b soit de 10 à 80% de 2a + b et toujours pourvu que le poids moléculaire moyen en nombre du surfactant non ionique méroxapol soit de 1.900 à 7.000.

16. Composition selon la revendication 15, dans laquelle la composition comporte en outre un sel de sulfite sélectionné parmi le groupe composé des sulfite de sodium, sulfite de potassium, sulfite de magnésium, sulfite de calcium, bisulfite de sodium, bisulfite de potassium, bisulfite de magnésium, bisulfite de calcium, métabisulfite de sodium, métabisulfite de potassium et métabisulfite de calcium.

17. Utilisation d'une composition de suspension aqueuse comprenant:
a) une quantité efficace sur le plan pharmaceutique de népafénac;
b) un surfactant non ionique poloxamère ou méroxapol dans une quantité de 0,001 à 0,15% (pds/v);
c) un agent de réglage de tonicité du glycol dans une quantité d'au moins 1,0% (pds/v) mais de moins que 4,0% (pds/v), et
d) de l'eau
dans laquelle la composition a une osmolalité de 150 à 500 mOsm/kg,
le surfactant non ionique poloxamère présente la formule 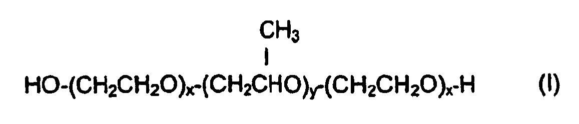 dans laquelle
x est 2 à 125 et y est 5 à 235, pourvu que 2x soit 10 à 80% de 2x + y, et toujours pourvu que le poids moléculaire moyen en nombre du surfactant non ionique poloxamère soit de 1.100 à 14.600;
et le surfactant non ionique méroxapol présente la formule 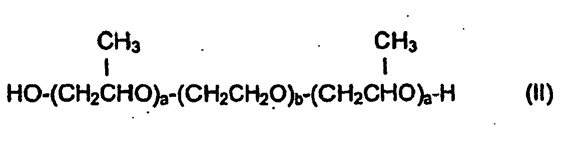 dans laquelle
a est 4 à 60 et b est 4 à 120, pourvu que b soit 10 à 80% de 2a + b, et toujours pourvu que le poids moléculaire moyen en nombre du surfactant non ionique méroxapol soit de 1.900 à 7.000,
l'agent de réglage de tonicité du glycol est sélectionné parmi le groupe composé des suivants: propylène glycol, glycérol, dipropylène glycol, diéthylène glycol, triéthylène glycol, 1,3-butylène glycol, 2,3-butylène glycol, 3-méthyl-1,3-butylène glycol, diglycérol, érythritol, pentaérythritol et néopentyl glycol,
pour la fabrication d'un médicament topique pour le traitement d'un trouble ophtalmique, dans lequel le trouble ophtalmique est sélectionné parmi le groupe des douleurs oculaires superficielles suivantes: l'uvéite, la sclérite, l'épisclérite, la kératopathie, une inflammation suite à de la chirurgie, l'endophtalmie, l'iritis, la dégénérescence maculaire atrophique, la rétinite pigmentaire, la rétinopathie iatrogénique, les larmes et les cavités rétiniennes, le syndrome d'Irvine, l'oedème maculaire diabétique, la rétinopahtie diabétique, la rétinopathie drépanocytaire, l'occlusion des veines et des artères rétiniennes, la neuropathie optique, la dégénérescence maculaire exsudative, le glaucome néovasculaire, la néovascularisation coméale, la cyclite, la rétinopathie drépanocytaire et le ptérygion.

18. Utilisation de la revendication 17, dans laquelle la composition comprend:
a) de 0,01 à 0,3% (pds/v) de népafénac;
b) de 0,001 à 0,15% (pds/v) du surfactant non ionique poloxamère ou méroxapol;
c) de 2,0 à 3,5% (pds/v) d'un agent de réglage de tonicité du glycol qui est sélectionné parmi le groupe composé des suivants: propylène glycol, glycérol et des mélanges de ceux-ci;
d) de 0,001 à 0,1% (pds/v) d'édétate disodium;
e) de 0,001 à 0,01% (pds/v) d'un conservateur acceptable sur le plan ophtalmique, et
f) de l'eau,
dans laquelle la composition présente un pH compris entre 7,5 et 8,0.
